# FASCICULE DE BREVET EUROPEEN

(11) **EP 0 538 085 B1**
(45) Date de publication et mention de la délivrance du brevet: **17.04.1996**
(21) Numéro de dépôt: 92402613.1
(22) Date de dépôt: 23.09.1992
(51) Int. Cl.: C07C 17/42, C07C 19/08

(54) **Stabilisation du 1,1-dichloro-1-fluoroéthane**
Stabilisierung von 1,1-Dichlor-1-fluorethan
Stabilisation of 1,1-dichloro-1-fluoroethane

(30) Priorité: 11.10.1991 FR 9112542
(43) Date de publication de la demande: 21.04.1993
(73) Titulaire: ELF ATOCHEM S.A., F-92800 Puteaux (FR)
(72) Inventeur: Lantz, André, F-69390 Vernaison (FR); Bertocchio, René, F-69390 Vourles par Vernaison (FR); Lambert, Patrick, F-69300 Caluire (FR)
(74) Mandataire: Leboulenger, Jean

(56) Documents cités:
- DE-A- 1 902 155
- DE-A- 3 434 886
- DE-B- 1 518 461
- US-A- 3 090 818
- CHEMICAL ABSTRACTS, vol. 111, no. 19, 6 Novembre 1989, Columbus, Ohio, US: abstract no. 173592h, page 675; & JP-A-01 050 829
- PATENT ABSTRACTS OF JAPAN, vol. 3, no. 79, (C-51)(86), 6 Juillet 1979, & JP-A-54 055 503
- PATENT ABSTACTS OF JAPAN, vol. 5, no. 54, (C-50)(726), 15 Avril 1981, & JP-A-56 005 422
- PATENT ABSTRACTS OF JAPAN, vol. 4, no. 30, (C-2)(512), 15 Mars 1980, & JP-A-55 007 207

## Description

La présente invention concerne la stabilisation du 1,1-dichloro-1-fluoroéthane. Ce composé, appelé aussi HFA 141b, a été proposé comme substitut aux chlorofluorocarbures (CFC), en particulier dans le domaine des agents d'expansion pour mousses de polymères, par exemple comme substitut au fluorotrichlorométhane (CFC 11), et dans celui des solvants comme substitut au 1,1,2-trichloro-1,2,2-trifluoroéthane (CFC 113).

Le HFA 141b peut être préparé par réaction du chlorure de vinylidène (désigné aussi par CV₂) ou du 1,1,1-trichloréthane avec de l'acide fluorhydrique. Ces procédés peuvent être réalisés soit en phase gaz, soit en phase liquide, en l'absence ou en présence de catalyseurs. De nombreux brevets concernant ces procédés de fabrication ont été publiés (US 2 894 044, EP 391 102 et EP 353 059 pour la fluoration phase gaz ; US 3 833 676 pour la fluoration phase liquide sans catalyseur ; EP 361 578, EP 378 942 et EP 391 103 pour la fluoration phase liquide en présence de catalyseur).

Tous ces procédés fournissent néanmoins un HFA 141b contenant de faibles quantités de CV₂. Ce CV₂ peut provenir d'une conversion incomplète dans le cas du procédé CV₂ + HF, mais dans le cas du procédé 1,1,1-trichloréthane + HF, il peut aussi provenir d'une décomposition du trichloréthane. Dans l'un et l'autre des deux procédés, le CV₂ peut aussi se former par décomposition du HFA 141b, en particulier au cours des traitements de finition tels que, par exemple, distillation, purification, séchage. Le chlorure de vinylidène peut être présent dans le HFA 141b à des concentrations de l'ordre de 200 ppm à 1 %.

D'autres impuretés éthylénique peuvent aussi être présentes dans le HFA 141b, comme le 1,1-chlorofluoroéthylène, mais en quantité beaucoup plus faible.

Le CV₂ et autres impuretés éthyléniques sont indésirables en quantités importantes dans l'utilisation du HFA 141b et les spécifications en CV₂ ou en mélange CCl₂ = CH₂ + CFCl = CH₂ ont été fixées à une valeur inférieure à 500 ppm.

Différents procédés ont été proposés pour réaliser l'élimination du CV₂ dans le HFA 141b. Ainsi, les brevets US 4 940 824 et 4 950 816 décrivent l'absorption sélective du CV₂ sur des tamis moléculaires carbonés et sur des charbons actifs ; cependant, les capacités d'absorption des deux absorbants ne sont pas très importantes et il est difficile de pouvoir descendre de façon économique à une teneur largement inférieure à 200 ppm.

D'autres procédés de purification ont aussi été décrits tels que l'élimination des impuretés éthyléniques par photochloration et/ou par réaction d'un hydracide (EP 401 493, EP 420 709 et US 4 962 244). Bien que ces procédés soient très efficaces, il est difficile d'éliminer entièrement toute trace de ces impuretés éthyléniques (en particulier de CV₂) et il serait intéressant de pouvoir commercialiser un HFA 141b contenant encore quelques centaines de ppm de CV₂, mais néanmoins stable.

Le HFA 141b pur est un produit parfaitement stable qui ne subit aucune transformation ou décomposition. Par contre, en présence de certains composés comme par exemple les alcools, le HFA 141b peut se décomposer ; le brevet US 4 816 174 décrit ainsi une stabilisation par le nitrométhane des mélanges HFA 141b-méthanol. En présence d'alcools ou de polyols, la plupart des chlorofluorocarbures ou hydrogénochlorofluorocarbures doivent aussi être stabilisés et de nombreux stabilisants ont ainsi été proposés tels que l'époxybutène (JP 01056630), l'α-méthylstyrène (JP 01050829), les esters acryliques ou méthacryliques (JP 01211538), le nitrométhane (JP 01128944), des mélanges de dérivés nitrés et d'époxydes (JP 01128945), des mélanges de dérivés du styrène et d'époxydes, de phénols, d'esters acryliques ou méthacryliques (JP 01056631, JP 01056632, JP 01211539).

Les brevets précités ne concernent en fait que la stabilisation des chlorofluorocarbures et hydrogénochlorofluorocarbures au cours de leur utilisation, tout particulièrement au cours de leur utilisation en tant qu'agents d'expansion de mousses plastiques. Il n'existe à notre connaissance aucun document concernant la stabilisation du HFA 141b lui-même.

La demanderesse a constaté qu'un HFA 141b contenant du CV₂ évolue lentement au cours du stockage. Cette instabilité se manifeste même pour du HFA 141b ne contenant que de faibles quantités de CV₂, c'est-à-dire environ 100 à 500 ppm ou même moins. L'évolution du HFA 141b se caractérise en particulier par une acidification du produit, mais la formation d'acide, tout particulièrement d'acide chlorhydrique, est aussi accompagnée de la formation d'autres produits tels que du phosgène et de produits peroxydiques. D'autres produits de décomposition ont aussi pu être mis en évidence, en particulier le formaldéhyde, l'acide formique, l'acide glyoxylique et l'acide monochloracétique. Bien que le mécanisme de cette décomposition ne soit pas connu, il est très vraisemblable qu'elle passe par une peroxydation du CV₂ par l'air ambiant ou dissous et que les différents produits détectés et signalés ci-dessus proviennent de la décomposition du peroxyde. Il n'est pas non plus possible de savoir si cette éventuelle peroxydation du CV₂ ne concerne que le CV₂ présent dans le HFA 141b ou si le HFA 141b n'est pas susceptible de se décomposer lui-même en CV₂ sous l'influence de ces produits de décomposition du même CV₂.

Cette évolution du HFA 141b au cours du stockage a été mise en évidence aussi bien à l'obscurité qu'à la lumière du jour, mais elle est beaucoup plus rapide à la lumière du jour ; le phénomène peut être considérablement accéléré par irradiation par une lampe UV.

Certains composés comme le nitrométhane ou le nitroéthane, qui ont été largement préconisés pour la stabilisation des chlorofluorocarbures et qui ont été revendiqués pour la stabilisation des mélanges HFA 141b-méthanol, n'ont absolument aucun effet sur la stabilisation du HFA 141b contenant du CV₂. Les dérivés phénoliques, en particulier l'éther monométhylique de l'hydroquinone (EMHQ) ne sont pas non plus très efficaces.

Il a maintenant été trouvé que l'addition d'hydrocarbures éthyléniques contenant au moins 4 atomes de carbone à un HFA 141b contenant des traces de CV₂ permet de réduire considérablement, voire d'inhiber entièrement cette réaction de décomposition et que ces hydrocarbures éthyléniques permettent de très bien stabiliser le HFA 141b.

Les hydrocarbures à utiliser selon l'invention peuvent comporter une ou plusieurs doubles liaisons éthyléniques et être des composés acycliques (linéaires ou ramifiés) ou cycliques (aromatiques ou non). Comme exemples non limitatifs d'hydrocarbures éthyléniques acycliques, on peut mentionner les alcènes tels que le 1- ou 2-butène, l'isobutylène, le 1-ou 2-pentène, l'amylène, le 2-méthyl-1-butène, le 1-hexène et le diisobutylène, les alcadiènes tels que le butadiène, l'isoprène, le 3-méthyl-1,2-butadiène et le 1,3-pentadiène, les alcatriènes tels que l'allo-ocimène. Comme exemples non limitatifs d'hydrocarbures éthyléniques cycliques, on peut mentionner les composés aromatiques tels que le styrène et l'α-méthylstyrène, ainsi que les composés cycloaliphatiques tels que le dipentène, les terpinènes et les pinènes.

Pour certaines applications du HFA 141b, il peut être avantageux d'utiliser comme stabilisant un hydrocarbure éthylénique ayant un point d'ébullition proche de celui du HFA 141b (32°C), en particulier un point d'ébullition compris entre environ 20 et 45°C.

La quantité nécessaire de stabilisant pour obtenir un HFA 141b parfaitement stable peut varier dans de larges limites. En général, une quantité de l'ordre de 100 à 1000 ppm de stabilisant est largement suffisante pour assurer une bonne stabilité et conservation du HFA 141b. Une quantité aussi importante n'est cependant pas toujours nécessaire et, dans certains cas, des teneurs de quelques parties par million (5 à 10 ppm) ou de quelques dizaines de ppm (10-100 ppm) est déjà suffisante.

Compte-tenu de cette faible quantité de stabilisant, le produit stabilisé peut être utilisé sans inconvénient à la place d'un HFA 141b parfaitement pur et non stabilisé, dans la plupart de ses applications.

Les essais et exemples suivants illustrent l'invention sans la limiter.

### ESSAI A

Un échantillon de 250 g de HFA 141b renfermant 2000 ppm de dichloro-1,1 éthylène (CV₂) est placé dans un flacon en pyrex et exposé à la lumière naturelle. Au bout de 18 jours, le produit est devenu acide et renferme des traces de peroxydes : 2 mg H₂O₂/litre (papier indicateur Peroxyde(e)-Test Merckoquant ^{R}10011). Apès 60 jours, le taux d'acidité atteint 8 méq H⁺/kg de HFA 141b.

### ESSAI B

Un échantillon de 100 ml de HFA 141b renfermant 300 ppm de CV₂ et 6 ppm de HFA 365 fmc (CF₃-CH₂-CF₂-CH₃) est placé dans un flacon en pyrex de 25 ml (diamètre : 27 mm) et exposé durant 5 heures au rayonnement d'une lampe UV haute pression type Hanovia 25 W (longueur d'onde max 360 nm), l'axe du flacon étant situé à 26 mm du bord de la lampe.

A l'issue de cette exposition, le produit est devenu acide (1,3 méq. H⁺/kg) et donne une réponse positive au test de recherche des peroxydes : 0,5 mg H₂O₂/litre. Un dosage de phosgène indique par ailleurs une teneur de 13 ppm.

### ESSAI C

Un échantillon de 10 ml du même HFA 141b qu'à l'essai B (300 ppm de CV₂ et 6 ppm de HFA 365 fmc) est additionné de 500 ppm de nitrométhane, puis traité dans les mêmes conditions qu'à l'essai B. Après exposition le produit est acide (3 méq. H⁺/kg) et contient des peroxydes correspondant à 1 mg H₂O₂/litre.

### EXEMPLE 1

Un échantillon de 10 ml du même HFA 141b qu'à l'essai B est additionné de 500 ppm d'α-méthylstyrène, puis exposé comme précédemment durant 5 heures au rayonnement de la lampe UV. A l'issue du test, le HFA 141b ne révèle aucune trace d'acidité (inférieure à 0,03 méq. H⁺/kg) et on ne détecte pas de peroxydes (teneur très inférieure au premier niveau de mesure du test, soit 0,5 mg H₂O₂/litre).

### EXEMPLE 2

On répète l'exemple précédent avec seulement 200 ppm d'α-méthylstyrène. méthylstyrène. Aucune trace d'acidité ou de peroxyde n'est détectée après 5 heures d'exposition aux rayonnements de la lampe.

### EXEMPLE 3

On répète l'exemple 1 en remplaçant l'α-méthylstyrène par la même quantité de 1,3-pentadiène (mélange cis et trans). Après le test, le produit est resté neutre et ne contient pas de peroxydes.

### EXEMPLES 4 A 9

On répète l'exemple 1, mais en remplaçant l'α-méthylstyrène par 50 ou 100 ppm de 1,3-pentadiène (mélange cis et trans), de 3-méthyl-1,2-butadiène ou de 2-méthyl-1-butène. Aucune trace d'acidité ou de peroxyde n'est détectée après 5 heures d'exposition aux rayonnements UV.

Le tableau suivant résume les essais et exemples précédents et leurs résultats.

| | **STABILISANT** | | **RESULTAT** | |
|---|---|---|---|---|
| | Nature | Concentration (ppm) | Acidité (méq.H⁺ /kg) | Peroxydes (mgH₂O₂/litre) |
| Essai B | (néant) | | 1,3 | 0,5 |
| Essai C | Nitrométhane | 500 | 3 | 1 |
| Exemple 1 | α-méthylstyrène | 500 | < 0,03 | non détectables (*) |
| Exemple 2 | α-méthylstyrène | 200 | < 0,03 | non détectables (*) |
| Exemple 3 | 1,3-pentadiène | 500 | < 0,03 | non détectables (*) |
| Exemple 4 | 1,3-pentadiène | 100 | < 0,03 | non détectables (*) |
| Exemple 5 | 1,3-pentadiène | 50 | < 0,03 | non détectables (*) |
| Exemple 6 | 3-méthyl-1,2-butadiène | 100 | < 0,03 | non détectables (*) |
| Exemple 7 | 3-méthyl-1,2-butadiène | 50 | < 0,03 | non détectables (*) |
| Exemple 8 | 2-méthyl-1-butène | 100 | < 0,03 | non détectables (*) |
| Exemple 9 | 2-méthyl-1-butène | 50 | < 0,03 | non détectables (*) |
| Exemple 10 | 2-méthyl-1,3-butadiène | 100 | < 0,03 | non détectables (*) |
| Exemple 11 | 2-méthyl-1,3-butadiène | 50 | < 0,03 | non détectables (*) |

| | | | | |
|---|---|---|---|---|
| (*) très nettement inférieur à 0,5 mg H₂O₂/litre. | | | | |

## Revendications

1. Procédé de stabilisation du 1,1-dichloro-1-fluoroéthane, contenant des traces de chlorure de vinylidène, caractérisé en ce qu'on lui ajoute une quantité suffisante d'au moins un hydrocarbure éthylénique contenant au moins 4 atomes de carbone.

2. Procédé selon la revendication 1, dans lequel la quantité d'hydrocarbure(s) éthylénique(s) est comprise entre 5 et 1000 ppm par rapport au poids de 1,1-dichloro-1-fluoroéthane, de préférence entre 10 et 100 ppm.

3. Procédé selon la revendication 1 ou 2, dans lequel l'hydrocarbure éthylénique est un alcène.

4. Procédé selon la revendication 1 ou 2, dans lequel l'hydrocarbure éthylénique est un alcadiène.

5. Procédé selon la revendication 1 ou 2, dans lequel l'hydrocarbure éthylénique est un composé aromatique, de préférence le styrène ou l'α-méthylstyrène.

6. Procédé selon la revendication 1 ou 2, dans lequel l'hydrocarbure éthylénique est un composé cyclique non aromatique.

7. Procédé selon l'une des revendications 1 à 4, dans lequel l'hydrocarbure éthylénique a un point d'ébullition compris entre 20 et 45°C.

8. Procédé selon la revendication 7, dans lequel l'hydrocarbure éthylénique est le 1,3-pentadiène, le 3-méthyl-1,2-butadiène, le 2-méthyl-1-butène, le 2-méthyl-1,3-butadiène ou un mélange de ces composés.

9. 1,1-Dichloro-1-fluoroéthane contenant de 5 à 1000 ppm d'au moins un hydrocarbure éthylénique tel que défini dans l'une des revendications 1 et 3 à 8.

## Claims

1. Process for stabilisation of 1,1-dichloro-1-fluoroethane containing traces of vinylidene chloride, characterised in that a sufficient quantity of at least one ethylenic hydrocarbon containing at least 4 carbon atoms is added thereto.

2. Process according to Claim 1, in which the quantity of ethylenic hydrocarbon(s) is between 5 and 1000 ppm in relation to the weight of 1,1-dichloro-1-fluoroethane, preferably between 10 and 100 ppm.

3. Process according to Claim 1 or 2, in which the ethylenic hydrocarbon is an alkene.

4. Process according to Claim 1 or 2, in which the ethylenic hydrocarbon is an alkadiene.

5. Process according to Claim 1 or 2, in which the ethylenic hydrocarbon is an aromatic compound, preferably styrene or α-methylstyrene.

6. Process according to Claim 1 or 2, in which the ethylenic hydrocarbon is a cyclic non-aromatic compound.

7. Process according to one of Claims 1 to 4, in which the ethylenic hydrocarbon has a boiling point between 20 and 45°C.

8. Process according to Claim 7, in which the ethylenic hydrocarbon is 1,3-pentadiene, 3-methyl-1,2-butadiene, 2-methyl-1-butene, 2-methyl-1,3-butadiene or a mixture of these compounds.

9. 1,1-Dichloro-1-fluoroethane containing 5 to 1000 ppm of at least one ethylenic hydrocarbon as defined in one of Claims 1 and 3 to 8.

## Patentansprüche

1. Verfahren zur Stabilisierung von 1,1-Dichlor-1-fluorethan, das Spuren Von Vinylidenchlorid enthält, dadurch gekennzeichnet, daß man zu diesem eine ausreichende Menge mindestens eines ethylenischen Kohlenwasserstoffs mit mindestens 4 Kohlenstoffatomen hinzugibt.

2. Verfahren nach Anspruch 1, wobei die Menge des/der ethylenischen Kohlenwasserstoffe(s) zwischen 5 und 1000 ppm, vorzugsweise zwischen 10 und 100 ppm, bezogen auf das Gewicht des 1,1-Dichlor-1-fluorethans, beträgt.

3. Verfahren nach Anspruch 1 oder 2, wobei der ethylenische Kohlenwasserstoff ein Alken ist.

4. Verfahren nach Anspruch 1 oder 2, wobei der ethylenische Kohlenwasserstoff ein Alkadien ist.

5. Verfahren nach Anspruch 1 oder 2, wobei der ethylenische Kohlenwasserstoff eine aromatische Verbindung, vorzugsweise Styrol oder α-Methylstyrol, ist.

6. Verfahren nach Anspruch 1 oder 2, wobei der ethylenische Kohlenwasserstoff eine cyclische nichtaromatische Verbindung ist.

7. Verfahren nach einem der Ansprüche 1 bis 4, wobei der ethylenische Kohlenwasserstoff einen Siedepunkt zwischen 20 und 45 °C besitzt.

8. Verfahren nach Anspruch 7, wobei der ethylenische Kohlenwasserstoff 1,3-Pentadien, 3-Methyl-1,2-butadien, 2-Methyl-1-buten, 2-Methyl-1,3-butadien oder eine Mischung dieser Verbindungen ist.

9. 1,1-Dichlor-1-fluorethan, enthaltend 5 bis 1000 ppm mindestens eines ethylenischen Kohlenwasserstoffs, wie er in den Ansprüchen 1 und 3 bis 8 definiert ist.
